# EUROPÄISCHE PATENTANMELDUNG

(11) **EP 1 892 674 A1**
(43) Veröffentlichungstag der Anmeldung: **27.02.2008**
(21) Anmeldenummer: 06017535.3
(22) Anmeldetag: 23.08.2006
(51) Int. Cl.: G07C 9/00, A61B 5/0482

(54) **Gehirnmusterbasiertes Zugangskontrollsystem**

(71) Anmelder: SIEMENS AKTIENGESELLSCHAFT, 80333 München (DE)
(72) Erfinder: Dinges, Clemens, 90587 Obermichelbach (DE); Schlereth, Michael, 91452 Wilhermsdorf (DE)

(57) **Zusammenfassung**

Die Erfindung beinhaltet ein Kontrollsystem (1) und ein Verfahren zur Zugangskontrolle eines Anwendungssystems (13), bei dem elektrisch messbare Daten infolge einer biometrischen Reaktion eines Benutzers (7) erfasst werden, wobei die biometrische Reaktion durch eine dem Benutzer (7) dargestellte Reizinformation ausgelöst wird. Ein Erfassungsmittel (2) zur Erfassung der elektrisch messbareren Daten, ein Bereitstellungsmittel (3) zur Bereitstellung von Referenzdaten und ein Vergleichsmittel (4) zum Datenvergleich sind im Kontrollsystem (1) vorgesehen. Die Referenzdaten werden bezüglich der Reizinformation und/oder der erfassten, elektrisch messbaren Daten bereitgestellt. Durch das Vergleichsmittel (4) werden die erfassten messbaren Daten mit den bereitgestellten Referenzdaten verglichen. Auf Basis des Vergleichsergebnisses erfolgt eine Berechtigung des Benutzers (7) zum Anwendungssystem (13). Durch dieses Kontrollsystem (1) ist es möglich, einen Benutzer dynamisch zu autorisieren, absolut zuverlässig zu erkennen und zweifellos zu authentifizieren.

## Beschreibung

Die Erfindung betrifft ein System zur Zugangskontrolle mit einem Erfassungsmittel zur Erfassung elektrisch messbarer Daten infolge einer biometrischen Reaktion eines Benutzers bzw. ein Verfahren zur Zugangskontrolle auf Basis von einer biometrischen Reaktion eines Benutzers.

Eine Person eindeutig zu identifizieren, authentifizieren und zu autorisieren, um ihr nur die ihr zugeordneten Rechte beispielsweise hinsichtlich der Bedienung einer Anlage oder dem Zugang zu einem System zu erteilen, nimmt in der heutigen Zeit dramatisch an Bedeutung zu. Die klassischen Zugangskontrollsysteme sind bei diesen erhöhten Anforderungen längst an ihre Grenzen gestoßen. Dabei basieren die Authentifizierung eines Benutzers auf Hardware-Komponenten (Schlüssel, Chipkarten usw.) bzw. auf Kennwortsystemen (Passwörter, PIN/TAN usw.). Sie lassen sich mit Hilfe krimineller Methoden, z.B. Entführung oder Erpressung von Schlüssel-Inhabern, Raub oder Duplizierung von Chipkarten, Datendiebstahl usw., relativ einfach überwinden, und können möglicherweise verloren oder vergessen werden. Deswegen kann solche Zugangskontrolle die erhöhten Sicherheitsanforderungen nicht erfüllen.

Neben der Authentifizierung spielt die Verwaltung der Autorisierung wegen der immer häufiger werdenden Wechslung von Personaleinsatz auch eine größere Rolle, obwohl die Verwaltung sehr aufwendig ist. Bisher erfolgt die Autorisierung von Personen für eine bestimmte Anwendung in der Regel durch eine ermächtigte Person (z.B. den Systemadministrator), der Berechtigungen erteilt. Die Zuteilung der Autorisierung dauert häufig lange, da ein Verwaltungs- und Genehmigungsweg eingehalten werden muss. Bei Aufgabenänderungen muss die erteilte Autorisation auch ggf. erneut geteilt werden.

Auch neuere, leistungsfähigere Ansätze beispielsweise auf der Basis biometrischer Methoden sind in der rauen Arbeitswirklichkeit des Produktionsprozesses oft nicht einsetzbar, da sie in der Regel eine saubere, büroähnliche Einsatzumgebung für die optischen bzw. elektrischen Erkennungsverfahren voraussetzen. Außerdem haben die bisher eingesetzten biometrischen Verfahren keine akzeptablen Fehlerquoten, insbesondere wenn nur ein biometrisches Verfahren in der Zugangskontrolle verwendet wird.

Der Erfindung liegt die Aufgabe zugrunde, die Treffsicherheit bei Zugangskontrollen zu erhöhen. Diese Aufgabe wird erfindungsgemäß durch ein Kontrollsystem mit den Merkmalen des Patentanspruchs 1 bzw. durch ein Verfahren mit den Merkmalen des Patentanspruchs 9 gelöst. Die abhängigen Ansprüche betreffen vorteilhafte Weiterbildungen und Ausgestaltungen des Systems bzw. des Verfahrens.

Der Erfindung liegt die Idee zugrunde, dass die mentale Aktivität einer Person von einer biometrischen Reaktion, insbesondere ihrer Gehirnströme, begleitet wird, wobei die biometrische Reaktion bei jedem Menschen unterschiedlich ausgeprägt ist. Dementsprechend wird ein Kontrollsystem zur Zugangskontrolle eines Anwendungssystems vorgesehen, wobei es ein Erfassungsmittel zur Erfassung elektrisch messbarer Daten eines Benutzers, ein Bereitstellungsmittel zur Bereitstellung von Referenzdaten und ein Vergleichsmittel zum Datenvergleich aufweist. Die elektrisch messbaren Daten sind die Daten, die infolge einer biometrischen Reaktion des Benutzers erzeugt werden, wobei diese biometrische Reaktion durch eine dem Benutzer dargestellte Reizinformation ausgelöst wird. Durch das Bereitstellungsmittel werden die Referenzdaten bezüglich der Reizinformation und/oder der erfassten, elektrisch messbaren Daten bereitgestellt. Dabei können die Referenzdaten personenbezogen bereitgestellt werden oder personenunabhängig sein. Durch das Vergleichsmittel werden die erfassten Daten mit den bereitgestellten Referenzdaten verglichen. Auf Basis des Vergleichsergebnisses erfolgt eine Berechtigung des Benutzers zum Anwendungssystem. Mit dem erfindungsgemäßen Kontrollsystem wird auch ein neues dynamisches Autorisierungsschema, nämlich eine so genannte Ad-hoc Autorisierung, ermöglicht. Die Rechtevergabe zum Anwendungssystem erfolgt dann erst zum Zeitpunkt der Autorisierungsanfrage, die abhängig vom Zustand des Benutzers ist. D.h. die Autorisierung kann nicht nur personenbezogen, sondern auch qualifikationsbezogen erfolgen, wobei die Qualifikation sich z.B. entweder auf die momentane Befindlichkeit wie Müdigkeit des Benutzers beziehen kann oder auf vorhandenes Know-how, nämlich die Handlungssicherheit des Benutzers. Dadurch ist es möglich, einen Benutzer absolut zuverlässig zu erkennen und zweifellos zu authentifizieren. Außerdem finden die unverwechselbaren biometrischen Kennzeichen Verwendung in der Sicherheitskontrolle bzw. Identifizierung, wobei evozierte elektrische Potentiale, wie z.B. EEG-Signale (electroencephalogram), zur treffsicheren und unumgehbaren Identifizierung und Authentifizierung sowie ggf. auch Autorisierung verwendet werden können.

Gemäß einer vorteilhaften Ausgestaltung der Erfindung weist das Kontrollsystem ein Mittel zur Darstellung der Reizinformation auf, damit die Reizinformation dem Benutzer präsentiert werden kann. Die Reizinformation kann z.B. ein optisches Muster (z.B. Piktogramm, Bilder, Videosequenz) bzw. ein akustisches Signal wie Geräusch, Sprache, Tonfolge oder Musiksequenz sein.

Weiterhin kann eine Gruppe von Mustern (Mustersets) in Kombination ebenfalls als Reizinformation verwendet werden. Durch die Präsentation der Mustersets sind die Benutzer von relevanten Gruppen beispielsweise so zu unterscheiden, dass die Benutzer mit den Mustersets teilweise bekannt bzw. unbekannt gemacht werden können. Durch diese so genannte Codierung, die die Benutzer mit den Mustersets teilweise bekannt und unbekannt machen, kann die Menge der unterschiedlichen Muster bzw. die Wahrscheinlichkeit einer Falschidentifizierung reduziert werden. Wenn man z.B. 10 Muster verwendet und daraus dem Benutzer 7 präsentiert, ergibt sich die Möglichkeit, 120 Benutzer zu codieren.

Vorteilhafterweise wird die Reizinformation einer Kennzeichnung oder Identifikation des Benutzers zugeordnet. Der Benutzer kann beim Kontrollsystem seine Kennzeichnung oder Identifikation eingeben. Somit kann die Reizinformation abhängig vom individuellen Benutzer präsentiert und zugeordnet werden.

Vorteilhafterweise werden die Referenzdaten durch die aktuell erfassten, elektrisch messbaren Daten ersetzt. Dadurch kann bei wiederholter Anwendung der Reizinformation ausgeglichen werden, dass die wiederholte Präsentation eines Reizes die mentale und damit die biometrische Reaktion, bspw. das EEG-Signal des Benutzers verändert.

Durch das Kontrollsystem kann ein Benutzer auch nur auf Basis seiner Erinnerung an die Reizinformation autorisiert werden. Dabei dient die Reizinformation nicht dazu, dass die Identifizierung des Benutzers authentifiziert wird, sondern eine einmalige Autorisierung der Reizinformation selbst zugeordnet ist. Die Reizinformation wird vor der Zugangskontrolle ausschließlich dem Benutzer, der zum Anwendungssystem berechtigt werden sollte, dargestellt. Diese Reizinformation wird nach deren Vergleich mit den entsprechenden Referenzdaten nicht mehr verwendet.

Gemäß einer weiteren vorteilhaften Ausgestaltung der Erfindung werden die elektrisch messbaren Daten auf Basis von einem mentalen Zustand des Benutzers erfasst. Die Reizinformation kann in diesem Fall durch die Umgebungssituation erfolgen. Dabei beurteilt das Kontrollsystem, welchen mentalen Zustand bzw. momentanen physischen und psychischen Zustand diese Situation beim Benutzer auslöst, und welches Know-how er besitzt. Dadurch kann die Autorisierung eines Benutzers dynamisch erfolgen. Ein Benutzer ist dann nur zu autorisieren, wenn er z.B. das entsprechende Know-how besitzt und mit dem System in einer bestimmten Lage zurechtkommt.

Vorteilhaftweise wird die biometrische Reaktion durch EEG (electroencephalogram), EOG (electrooculogram) oder EMG (electromyogram) gemessen. Bei solchen Verfahren können evozierte elektrische Potentiale als unveränderliche Eigenschaften von Menschen gemessen werden. Durch z.B. EEG-Abnahme kann festgestellt werden, ob dem Benutzer der Reiz bekannt ist.

Dem oben dargestellten Kontrollsystem entsprechend wird ein Verfahren zur Zugangskontrolle eines Anwendungssystems angegeben, um einen Benutzer zuverlässig zu erkennen und zweifellos zu authentifizieren. Bei diesem Verfahren werden elektrisch messbare Daten infolge einer biometrischen Reaktion des Benutzers erfasst, wobei die biometrische Reaktion durch eine dem Benutzer dargestellte Reizinformation ausgelöst wird. Bezüglich der Reizinformation und/oder der erfassten, elektrisch messbaren Daten werden Referenzdaten bereitgestellt werden. Die erfassten messbaren Daten werden mit den bereitgestellten Referenzdaten verglichen werden, und auf Basis des Vergleichsergebnisses erfolgt eine Berechtigung des Benutzers zum Anwendungssystem.

Im Folgenden wird die Erfindung anhand der in den Figuren dargestellten Ausführungsbeispiele näher beschrieben und erläutert. Es zeigen:
- FIG 1: eine erfindungsgemäße Zugangskontrolle auf Basis personenbezogener Aufzeichnungen,
- FIG 2: eine erfindungsgemäße Zugangskontrolle für ein anonyme Autorisierung,
- FIG 3: eine erfindungsgemäße Zugangskontrolle auf Basis von situationsspezifischen mentalen Zuständen.

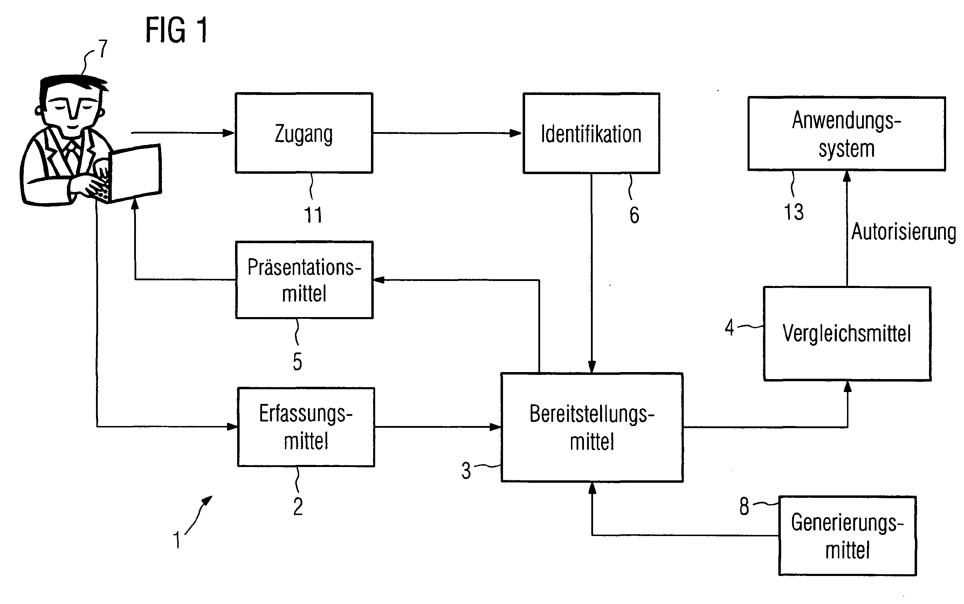

In FIG 1 wird eine gehirnmusterbasierte Zugangskontrolle eines Anwendungssystems 13 gemäß eines Kontrollsystems 1 gezeigt, wobei eine Authentifizierung auf Basis einer personenbezogenen Aufzeichnung erfolgt. Im Kontrollsystem 1 sind ein Erfassungsmittel 2 zur Erfassung elektrisch messbarer Daten eines Benutzers 7, ein Bereitstellungsmittel 3 zur Bereitstellung von Referenzdaten und ein Vergleichsmittel 4 zum Datenvergleich vorgesehen. Die elektrisch messbaren Daten werden infolge einer biometrischen Reaktion des Benutzers 7 erzeugt, wobei diese biometrische Reaktion durch eine dem Benutzer 7 dargestellte Reizinformation ausgelöst wird. Die biometrische Reaktion ist die Gehirnreaktion des Benutzers 7 und wird durch EEG gemessen. Darüber hinaus werden ein Präsentationsmittel 5 zum Erzeugen des Reizes und ein Generierungsmittel 8 zur Generierung des Reizes im Kontrollsystem 1 vorgesehen. Dabei kann der Reiz auch eine Gruppe von Mustern (Mustersets) sein.

Die in FIG 1 dargestellte Zugangskontrolle erfolgt in zwei Schritten, nämlich die Konditionierung und die Authentifizierung. Zugeordnet zur Authentifizierung ist auch eine Autorisierung möglich. Zur Bereitstellung der erforderlichen Referenzdaten bzw. Identifikation 6 für einen Benutzer 7 wird die Konditionierung vor der Authentifizierung durchgeführt. Dem generierten Reiz wird bzgl. der Identifikation 6 durch das Bereitstellungsmittel 3 eine Benutzerinformation vom Benutzer 7 zugeordnet. Zur Konditionierung des Benutzers wird dem Benutzer der generierte Reiz über das Präsentationsmittel 5 übermittelt. Eine infolge des Reizes ausgelöste biometrische Reaktion - z.B. die elektrische Aktivität von Gehirn (Gehirnmuster) oder ggf. in Kombination mit weiteren biometrischen Merkmalen - des Benutzers 7 wird durch das Erfassungsmittel 2 gemessen und als Referenzdaten aufgezeichnet und dem Benutzer 7 zugeordnet. Die entsprechenden Referenzdaten werden durch das Bereitstellungsmittel 3 auf Basis von diesem Reiz bzw. von den erfassten Gehirnmustern bereitgestellt. Hierbei können die Daten der Identifikation 6 des Benutzers 7 den Referenzdaten zugeordnet werden. Es ist auch möglich, den Referenzdaten einen mentalen Zustand (z.B. Handlungssicherheit und Müdigkeit, usw.) des Benutzers 7 zu zuordnen. In der Regel werden die Referenzdaten mit den Identifizierungsdaten und dem generierten Reiz zusammen in einer Datenbank hinterlegt.

Wenn der Benutzer 7 nach der Konditionierung über den Zugang 11 zum Anwendungssystem 13, z.B. einem geschützten Bereich (Anwendung, Raum), möchte, gibt er gegenüber dem Kontrollsystem 1 seine Identifikation 6 an. Anschließend wird ihm der seiner Identifikation 6 zugeordnete Reiz durch das Präsentationsmittel 5 aus der Konditionierungsphase präsentiert. Mittels des Vergleichsmittels 4 vergleicht das Kontrollsystem 1 die hinsichtlich der Gehirnmuster des Benutzers 7 erfassten Daten mit den entsprechenden Referenzdaten, die im Konditionierungsschritt erfasst wurden, und überprüft, ob die Reaktion in einem bestimmten Toleranzbereich liegt. Nach dem Vergleichsergebnis kann die Berechtigung des Benutzers 7 erfolgen. Liegt das Vergleichsergebnis oberhalb einer festgelegten Akzeptanzschwelle, lautet die Entscheidung des Kontrollsystems 1 passend und akzeptiert das Kontrollsystem 1 den Benutzer 7, andernfalls lautet die Entscheidung unpassend und der Benutzer 7 wird zurückgewiesen. Bei positiver Entscheidung werden die erfassten Daten als Referenzdaten neu abgespeichert, dadurch kann bei wiederholter Anwendung ausgeglichen werden, dass die wiederholte Präsentation eines Reizes die mentale Reaktion verändert.

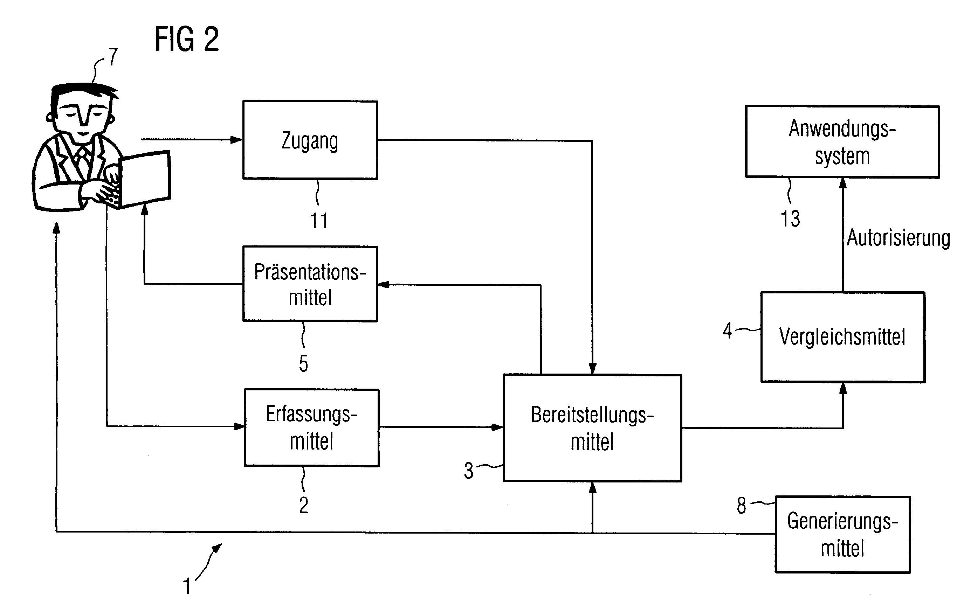

FIG 2 zeigt eine andere Ausführungsform des Kontrollsystems 1. Diese Art der Autorisierung ist nicht zwangsweise personengebunden und auch nicht auf eine Person beschränkt. Wie aus FIG 2 ersichtlich ist, wird dem generierten Reiz keine Benutzerinformation zugeordnet, denn der Benutzer muss dem Kontrollsystem 1 seine Identifikationsdaten (Kennwort, Benutzname) nicht mitteilen. Dem Beispiel in FIG 1 entsprechend erfolgt die Zugangskontrolle auch in zwei Schritten, nämlich die Konditionierung und die Autorisierung. Die diesseitige Konditionierung ist eine anonyme Konditionierung, wobei der Reiz nicht dazu dient, den Benutzer 7 zu authentifizieren, sondern dem Reiz selbst eine einmalige Autorisierung zugeordnet ist. Wenn der Reiz einer nicht autorisierten Person präsentiert wurde, hat eine autorisierte Person über diesen Reiz keinen Systemzugang mehr. Deshalb könnte der Reiz z.B. erst nach einer speziellen Benutzeranforderung an ihn präsentiert werden. Eine derartige Konditionierung kann auch außerhalb des Kontrollsystems 1, z.B. an einem herkömmlichen Computer über das Internet erfolgen. Der Reiz kann z.B. einer beliebigen Person als Bild per Post zugeschickt werden.

Sinnvoll ist es vor allem dann, wenn die autorisierte Person selbst verhindern möchte, dass jemand außer ihr die Autorisierung nutzen kann. Es ist wichtig darauf zu achten, dass vor der Autorisierung nur den Benutzer 7, der autorisiert werden soll, dem Reiz ausgesetzt ist. Deshalb sollte das Generierungsmittel 8 in der Lage sein, solche Reize zu erzeugen, wobei den Reizen mit hoher Wahrscheinlichkeit bisher noch kein möglicher Benutzer ausgesetzt war, und sie aber trotzdem einen hohen Wiedererkennungswert besitzen. Dieses Bedürfnis kann auch über die Kombination von mehreren gleichartigen oder verschiedenartigen Reizen erfüllt werden.

Wenn der Benutzer 7 über den Zugang 11 zum Anwendungssystem 13 möchte, wird der Reiz bei Zugangskontrolle präsentiert. Durch eine Erinnerung an den angezeigten Reiz können biometrische Reaktionen des Benutzers 7 ausgelöst werden. Aus der mentalen Reaktion des Benutzers 7 kann festgestellt werden, ob er den Reiz gekannt hat, oder nicht. Ist der Reiz dem Benutzer 7 bekannt, erfolgt eine Autorisierung für ihn direkt. Da die Präsentation des Reizes unabhängig von individuellem Benutzer ist, kann der Reiz einer Person, die nicht berechtigt werden soll, bekannt sein. Somit kann der Reiz wird nach dem ersten Versuch nicht mehr verwendet werden.

Ein wichtiger Unterschied zur Ausführung in FIG 1 besteht darin, dass beim System in FIG 1 die Authentizität des Benutzers geprüft wird (an die eine Autorisierung gekoppelt) während im System in FIG 2 der Reiz direkt der Autorisierung des Benutzers zugeordnet werden könnte, ohne dass die Identität des Benutzers bekannt ist (anonyme Autorisierung).

Die in FIG 2 dargestellte Ausführungsform kann auch so verwendet werden, um z.B. durch Zugangskontrollen an Flughäfen, Hochhäusern oder anderen gefährdeten Orten die regelmäßig zu erstellenden "Sicherheitsrisikoprofile" aller Menschen zu erfassen. Durch EEG-Abnahme kann festgestellt werden, ob jemanden Begriffe, Szenen oder andere Dinge bekannt sind.

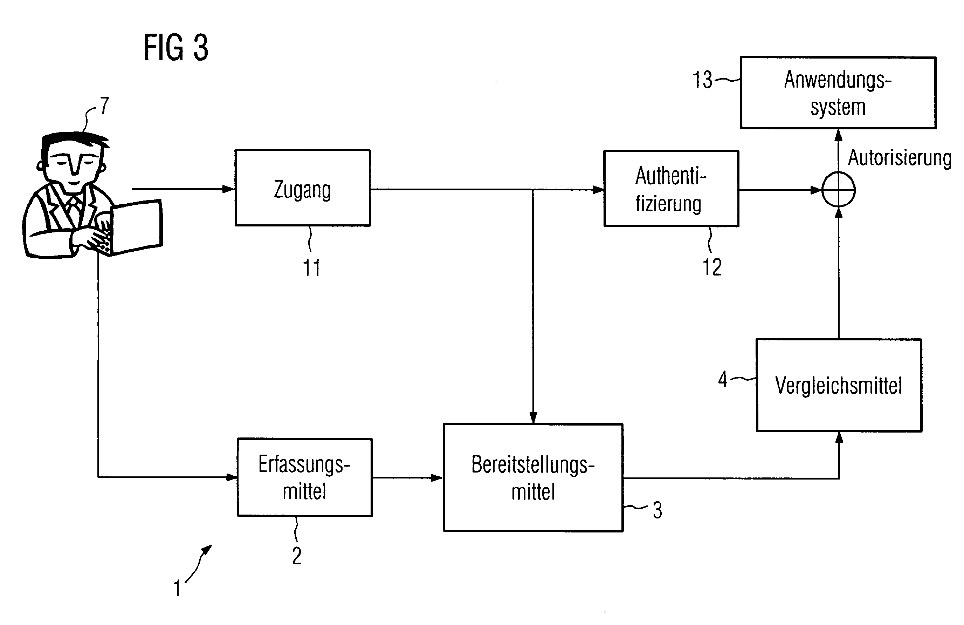

Gegenüber den oben genannten zwei Ausführungsformen zeigt FIG 3 eine Zugangskontrolle auf Basis situationsspezifischer mentaler Zustände von Benutzern. In diesem Fall braucht man keine Konditionierung. Die Autorisierung erfolgt direkt aus Zuständen eines Benutzers 7 heraus, wobei die Zustände die mentale Fitness bzw. der Ausbildungsgrad des Benutzers 7 sein können. Deswegen erfolgt der Reiz durch die Umgebungssituation, z.B. eine Störungssituation. Das Kontrollsystem 1 beurteilt, welchen mentalen Zustand diese Situation beim Benutzer 7 auslöst, z.B. Verwirrung, Selbstbewusstsein, Sicherheit/Unsicherheit. Obwohl der Benutzer 7 beim Zugang 11 authentifizierbar ist, bekommt er jedoch nach der erfolgreichen Authentifizierung 12 nicht automatisch alle zugewiesenen Zugangsrechte. Es wird überprüft, ob er auch mental in der Lage ist, diese Rechte zum aktuellen Zeitpunkt auszuüben und damit die Aufgabe zu lösen. Auf Basis dieser Beurteilung kann der Benutzer 7 autorisiert werden. Die Autorisierung erfolgt also dynamisch nach dem Know-how und dem momentanen physischen und psychischen Zustand des Benutzers 7.

Als ein Beispiel wäre zu nennen, dass der Benutzer 7 jedoch kein Zugriffsrecht auf eine bestimmte Maschine/Anlage des Anwendungssystems 13 hat, auch wenn er bereits für das Anwendungssystem 13 authentifiziert/identifiziert wurde. Falls eine Störung auftritt, kann das Kontrollsystem 1 aufgrund des mentalen Zustands des Benutzers 7 entscheiden, ob er die Störungssituation erkennt und ob er von seinem Wissensstand der Aufgabe gewachsen ist. Nach dem Ergebnis dieser Entscheidung darf der Benutzer 7 die Störung beheben oder muss er einen Kollegen zu Hilfe hohlen.

In Notfall ist es auch möglich, dass eine Person, die die Störung bebeben kann, nach dem Entscheidungsergebnis direkt ein Zugangsrecht zum Anwendungssystem 13 hat, auch wenn sie vorher nicht als Benutzer des Anwendungssystems 13 anerkannt wurde. Dieses Szenario ist beispielsweise vor allem bei Einsatz von Fremdpersonal für Serviceaufgabe (Wartung, Putzen, usw.) vorteilhaft. Auch wenn das Kennwort des Benutzers 7 von jemandem geklaut wurde, kann das Kontrollsystem 1 feststellen, ob eine Person tatsächlich diejenige ist, die sie behauptet hat.

## Patentansprüche

1. Kontrollsystem (1) zur Zugangskontrolle eines Anwendungssystems (13), mit einem Erfassungsmittel (2) zur Erfassung elektrisch messbarer Daten infolge einer biometrischen Reaktion eines Benutzers (7), wobei die biometrische Reaktion des Benutzers (7) durch eine ihm dargestellte Reizinformation auslösbar ist, mit einem Bereitstellungsmittel (3) zur Bereitstellung von Referenzdaten bezüglich der Reizinformation und/oder der erfassten, elektrisch messbaren Daten, und mit einem Vergleichsmittel (4) zum Vergleich der erfassten Daten mit den bereitgestellten Referenzdaten, wobei eine Berechtigung des Benutzers (7) zum Anwendungssystem (13) auf Basis des Vergleichsergebnis erfolgt.

2. Kontrollsystem (1) nach Anspruch 1,
**dadurch gekennzeichnet, dass**
das Kontrollsystem (1) ein Mittel (8) zur Generierung von Reizinformation aufweist.

3. Kontrollsystem (1) nach Anspruch 1 oder 2,
**dadurch gekennzeichnet, dass**
das Kontrollsystem (1) ein Mittel (5) zur Darstellung Präsentation der Reizinformation aufweist.

4. Kontrollsystem (1) nach einem der Ansprüche 1 bis 3,
**dadurch gekennzeichnet, dass**
die Reizinformation eine Gruppe von Mustern ist.

5. Kontrollsystem (1) nach einem der Ansprüche 1 bis 4,
**dadurch gekennzeichnet, dass**
die Reizinformation einer Kennzeichnung/Identifikation 6 des Benutzers (7) zuordenbar ist.

6. Kontrollsystem (1) nach einem der vorherigen Ansprüche,
**dadurch gekennzeichnet, dass**
die Referenzdaten durch die aktuell erfassten, elektrisch messbaren Daten ersetzbar sind.

7. Kontrollsystem (1) nach einem der Ansprüche 1 bis 4,
**dadurch gekennzeichnet, dass**
die Reizinformation ausschließlich dem Benutzer (7) darstellbar ist.

8. Kontrollsystem (1) nach einem der Ansprüche 1 bis 4,
**dadurch gekennzeichnet, dass**
die elektrisch messbaren Daten auf Basis von einem mentalen Zustand des Benutzers (7) erfassbar sind.

9. Kontrollsystem (1) nach einem der vorherigen Ansprüche,
**dadurch gekennzeichnet, dass**
die biometrische Reaktion des Benutzers (7) durch EEG (electroencephalogram), EOG (electrooculogram) oder EMG (electromyogram) messbar ist.

10. Verfahren zur Zugangskontrolle eines Anwendungssystems (13), bei dem elektrisch messbare Daten infolge einer biometrischen Reaktion eines Benutzers (7) erfasst werden, wobei die biometrische Reaktion des Benutzers (7) durch eine ihm dargestellte Reizinformation ausgelöst wird, wobei Referenzdaten bezüglich der Reizinformation und/oder der erfassten, elektrisch messbaren Daten bereitgestellt werden, wobei die erfassten Daten mit den bereitgestellten Referenzdaten verglichen werden, und wobei eine Berechtigung des Benutzers (7) zum Anwendungssystem (13) auf Basis des Vergleichsergebnis erfolgt.

11. Verfahren nach Anspruch 10,
**dadurch gekennzeichnet, dass**
die Reizinformation als eine Gruppe von Mustern vorgesehen wird.

12. Verfahren nach Anspruch 10 oder 11,
**dadurch gekennzeichnet, dass**
die Reizinformation einer Kennzeichnung/Identifikation 6 des Benutzers (7) zugeordnet wird.

13. Verfahren nach einem der Ansprüche 10 bis 12,
**dadurch gekennzeichnet, dass**
die Referenzdaten durch die aktuell erfassten, elektrisch messbaren Daten ersetzt werden.

14. Verfahren nach einem der Ansprüche 10 bis 12,
**dadurch gekennzeichnet, dass**
die Reizinformation ausschließlich dem Benutzer (7) dargestellt wird.

15. Verfahren nach einem der Ansprüche 10 bis 12,
**dadurch gekennzeichnet, dass**
die elektrisch messbaren Daten auf Basis von einem mentalen Zustand des Benutzers (7) erfasst werden.

16. Verfahren nach einem der Ansprüche 10 bis 15,
**dadurch gekennzeichnet, dass**
die biometrische Reaktion des Benutzers (7) in Form von EEG (electroencephalogram), EOG (electrooculogram) oder EMG (electromyogram) gemessen wird.
